# EUROPEAN PATENT SPECIFICATION

(11) **EP 4 704 962 B1**
(45) Date of publication and mention of the grant of the patent: **02.12.2026**
(21) Application number: 24723983.3
(22) Date of filing: 29.04.2024
(51) Int. Cl.: A61N 1/04, A61N 1/36

(54) **TRANSCUTANEOUS AURICULAR VAGUS NERVE STIMULATION**
TRANSKUTANE AURIKULÄRE STIMULATION DES VAGUSNERVS
STIMULATION TRANSCUTANÉE DU NERF VAGUE AURICULAIRE

(30) Priority: 02.05.2023 SE 2350523
(43) Date of publication of application: 11.03.2026
(73) Proprietor: Cymbathera AB, 223 63 Lund (SE)
(72) Inventor: ANDERSSON, Ulf, 131 50 Saltsjö-Duvnäs (SE); BRORSSON, Göran, 224 66 Lund (SE)
(74) Representative: Barker Brettell Sweden AB
(86) International application number: PCT/SE2024/050411
(87) International publication number: WO 2024/228655

(56) References cited:
- WO-A2-2022/221858
- US-A1- 2019 351 230
- US-A1- 2021 187 296
- US-A1- 2022 143 390

## Description

### TECHNICAL FIELD

The present invention generally relates to transcutaneous auricular vagus nerve stimulation (taVNS).

### BACKGROUND

The vagus nerves play a major role in maintaining autonomic tone throughout the brain, thorax and abdomen. Electrical vagus nerve stimulation (VNS) is currently authorized for drug-resistant epilepsy, major depression and morbid obesity. Such VNS is conducted in the form of a cervical implantable device. VNS implantation requires a surgical procedure that positions the lead wire at the cervical portion of the trunk of the left vagus nerve while the pulse generator is inserted subcutaneously in a pocket created in the upper chest. The most common adverse effects of implanted VNS devices are related to the surgical intervention at implantation. Laryngo-tracheal dysfunction, which is related to the stimulation of the inferior recurrent laryngeal nerve, occurs in approximately two thirds of cohorts treated for epilepsy and is usually transient. But there are also other surgical challenges including electrode fracture, dislocation, generator malfunction, wound infection, recurrent laryngeal nerve palsy and cardiac arrhythmias under test stimulation.

An attractive and promising approach to circumvent the caveats of invasive VNS in humans is to use VNS engendered by an external pulse generator. Two types of transcutaneous VNS have been developed: transcutaneous cervical VNS (tcVNS) and transcutaneous auricular VNS (taVNS). tcVNS can be accomplished by delivering an electrical signal to the cervical vagus nerve, situated within the carotid sheath posterolateral to the internal and common carotid arteries and medial to the internal jugular vein, with electrodes placed on the skin covering the sternocleidomastoid muscle. Although implanted electrodes for VNS are positioned at a similar location, the position of the vagus nerve beneath the skin, superficial fascia and sternocleidomastoid muscle makes the transcutaneous electrical stimulation of the vagus fibers difficult to access with current bioelectronic devices most likely stimulating both afferent and efferent fibers in the vagus nerve bundle.

The auricular branch of the vagus nerve projects sensory input to the brain stem nucleus tractus solitarius (NTS), which receives approximately 95% of all vagal afferents. The NTS projects to numerous areas in forebrain, amygdala, hippocampal, limbic and brainstem structures including the nucleus ambiguus, the locus coeruleus and the dorsal motor nucleus, which promotes motor outflow of the vagus nerve. Functional magnetic resonance imaging (fMRI) studies in humans verify that the central projections of the auricular branch of the vagus nerve are consistent with the vagal projections activated after invasive VNS and can be accessed non-invasively via the external ear in taVNS. taVNS, generally performed at home, is accomplished using a small battery-powered hand-held stimulating device generating pulsed electrical currents, which are delivered via skin electrodes in the auricle.

Shortcomings of these handheld taVNS devices is to accurately deliver the stimulation pulses at correct positions in the auricle, and provide sufficient electrical contact between the stimulation electrodes and the skin during the complete stimulation session.

US 2022/0143390 discloses a multi-electrode ear shell including an inner surface and an outer surface. The inner surface corresponds to a surface of an ear and is configured to overlap a cymba and a cavum of the ear. The multi-electrode ear shell further includes multiple sockets configured to receive stimulation electrodes that can be in contact to various locations including the cymba, cavum, crus of helix, antihelix, tragus, auricular acupuncture points, and the like. A conductive gel is applied on the skin of the ear to provide sufficient electrical contact between the stimulation electrodes and the skin.

There is still a need for a user-friendly taVNS system that provides efficient taVNS stimulation.

### SUMMARY

It is a general objective to provide a user-friendly transcutaneous auricular vagus nerve stimulation (taVNS) system that provides efficient taVNS stimulation.

This and other objectives are met by embodiments as disclosed herein.

The present invention is defined by the independent claims. Further embodiments of the invention are defined by the dependent claims.

An aspect of the invention relates to a taVNS system comprising an electrode support, a cymba concha electrode attached to and protruding from the electrode support and configured to be in contact with skin in cymba concha of an ear of a human subject, and a cavum concha electrode attached to and protruding from the electrode support and configured to be in contact with skin in cavum concha of the ear of the human subject. The taVNS system also comprises an elastic headband attached to the electrode support and configured, when attached to a head of the human subject, to exert a pressure onto the electrode support to press the cymba concha electrode towards the skin in cymba concha of the ear of the human subject and the cavum concha electrode onto the skin in cavum concha of the ear of the human subject. According to the invention, the electrode support does not comprise any other electrodes than the cymba concha electrode and the cavum concha electrode. One of the cymba concha electrode and the cavum concha electrode is adjustably attached to the electrode support and protrudes at an adjustable distance from the electrode support and the other of the cymba concha electrode and the cavum concha electrode is fixedly attached to the electrode support and protrudes at a fixed distance from the electrode support

The taVNS system of the invention is user friendly allowing it to be used at home without the guidance of any physician. The taVNS system is constructed to reliably and reproducibly provide efficient vagus nerve stimulation at each stimulation session by correctly aligning the electrodes at the intended stimulation sites in the external ear. An efficient vagus nerve stimulation is further achieved without the need for any contact promoting medium, such as electrically conductive gels.

### BRIEF DESCRIPTION OF THE DRAWINGS

The embodiments, together with further objects and advantages thereof, may best be understood by making reference to the following description taken together with the accompanying drawings, in which:
Figs. 1A-1C illustrate a taVNS system according to an embodiment;
Fig. 2 illustrates a taVNS system according to another embodiment;
Fig. 3 illustrates a taVNS system according to a further embodiment;
Fig. 4 illustrates a stimulation device that can be used in a taVNS system according to the embodiments;
Fig. 5 is a flow chart illustrating a method for taVNS in a human subject; and
Figs. 6A-6E illustrates cytokine levels in lipopolysaccharide (LPS) activated whole blood cultures from 12 healthy donors before and after taVNS treatment.

### DETAILED DESCRIPTION

The present invention generally relates to transcutaneous auricular vagus nerve stimulation (taVNS), and in particular to a system for taVNS.

The taVNS system of the invention is designed to facilitate effective and reproducible transcutaneous auricular vagus nerve stimulation of a human subject, for instance, at home. This is achieved by placement of the stimulation electrodes at the correct positions in the external ear of the human subject. Such a correct electrode positioning is currently hard to achieve in a reproducible way by the human subject using handheld taVNS stimulating devices. Hence, it is not uncommon with such handheld taVNS stimulating devices that the human subject is not able to correctly position one or both of the stimulating electrodes at the intended stimulation sites in the external ear, with a consequence of less effective stimulation delivery and treatment.

A further shortcoming with prior art taVNS stimulating devices, including the stimulation system as disclosed in US 2022/0143390, is that an electrically conductive gel needs to be applied to the skin of the external ear to provide sufficient electrical contact between the stimulation electrodes and the skin surface. Such electrically conductive gels are often perceived as uncomfortable by the human subjects, and may cause skin irritation in particular during prolonged use.

The stimulating system disclosed in US 2022/0143390 is further marred by requiring design of a subject-specific multi-electrode ear shell for each individual human subject to be treated. Firstly, the clinician needs to take three-dimensional (3D) images of the external ear of each human subject and then generate the multi-electrode ear shell from such 3D images. Although, the multi-electrode ear shell is produced for a specific human subject, it is still cumbersome to securely insert in the external ear while minimizing movement of the multi-electrode ear shell relative the external ear during stimulation. The reason being that the multi-electrode ear shell needs to contain a plurality of protrusions and recesses to accommodate specific regions of the external ear, such the stem of antihelix, inferior crus of antihelix, superior crus of antihelix and antitragus. It is often hard for the human subject to correctly align the protrusions and recesses correctly with the specific regions or structures of the external ear each and every time the transcutaneous auricular vagus nerve stimulation should take place.

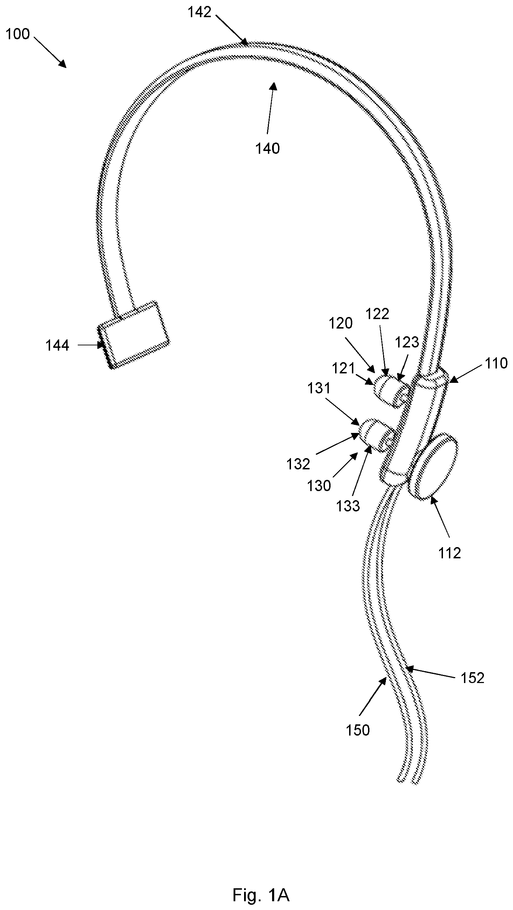

The present invention relates to a taVNS system 100, see Figs. 1A-1C, 2 and 3. The taVNS system 100 comprises an electrode support 110, a cymba concha electrode 120, a cavum concha electrode 130 and an elastic headband 140. The cymba concha electrode 120 and the cavum concha electrode 130 are attached to and protrude from the electrode support 110. This cymba concha electrode 120 is configured to be in contact with skin in cymba concha of an ear of a human subject. The cavum concha electrode 130 is correspondingly configured to be in contact with skin in cavum concha of the ear of the human subject. The elastic headband 140 is attached to the electrode support 110 and is configured, when attached to a head of the human subject, to exert a pressure onto the electrode support 110 to press the cymba concha electrode 120 towards the skin in cymba concha of the ear of the human subject and the cavum concha electrode 130 onto the skin in cavum concha of the ear of the human subject.

The electrode support 110 of the taVNS system 100 does not comprise any other electrodes than the cymba concha electrode 120 and the cavum concha electrode 130.

The electrode support 110 of the taVNS system 100 thereby only contains two electrodes, i.e., the cymba concha electrode 120 and the cavum concha electrode 130. The design of the taVNS system 100 of the invention enables a correct positioning of these electrodes 120, 130 in contact with the skin at the cymba concha and cavum concha, respectively, of the ear. This should be compared to the stimulating system of US 2022/0143390, in which the multi-electrode ear shell contains a plurality of electrodes to, in theory, be capable of stimulating a plurality of sites, such as the superior crus of antihelix, helix, scapha, the stem of antihelix, the concha, antitragus, the ear lobe, intertragal notch, tragus, crus of helix, inferior crus of antihelix and triangular fossa. Such a plurality of potential stimulation sites may be necessary for the stimulating system of US 2022/0143390 in order to provide the intended electrical stimulation to the target site due to above-mentioned problems of correctly attaching the multi-electrode ear shell to the external ear.

Experimental data presented herein shows that the electrode support 110 with attached and protruding cymba concha electrode 120 and cavum concha electrode 130 achieves an efficient taVNS capable of inducing a biological effect in the treated human subjects with merely two such electrodes 120, 130 attached to and protruding from the electrode support 110.

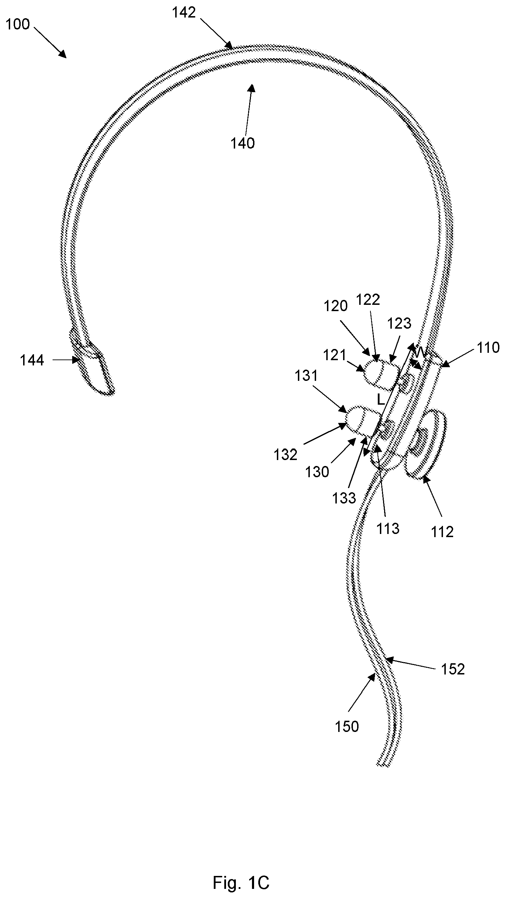

Furthermore, the positions of the two electrodes 120, 130 at the electrode support 110 is preferably fixed in terms of not being adjustable along the length L or width W of the electrode support 110, see Fig. 1C. However, the protrusion of one or both electrodes 120, 130 from the electrode support 110 is preferably adjustable as will be further described herein. This enables a less complex design of the electrode support 110 as compared to the multi-electrode ear shell of US 2022/0143390 and still achieving efficient taVNS. A further benefit is that the taVNS system 100 of the invention does not need to be subject-specific, i.e., one and the same taVNS system 100 could be used to provide taVNS to multiple different human subjects.

The cymba concha electrode 120 and the cavum concha electrode 130 are made of an electrically conducting material, preferably an electrically conducting metal or metal alloy. The electrically conducting material, preferably electrically conducting metal or metal alloy, should not only have good electrical conductivity but should also preferably be biologically acceptable. Electrically conducting metals and metal alloys generally have a resistivity at 20°C in the order of 10⁻⁸ Ωm and thereby an electrical conductivity at 20°C in the order of 10⁶-10⁷ S/m. In a particular embodiment, the electrically conducting material, preferably electrically conducting metal or metal alloy, of the electrodes 120, 130 has an electrical conductivity of at least 10⁶ S/m, preferably at least 0.5×10⁷ S/m, and more preferably at least 10⁷ S/m at 20°C. More preferably, the electrically conducting material, preferably electrically conducting metal or metal alloy, has an electrical conductivity at 20°C of at least 3×10⁷ S/m.

Biologically acceptable or biologically tolerant as used herein indicates that the electrically conducting material, preferably electrically conducting metal or metal alloy, does not cause any significant deleterious effects to the skin in the ear when the electrodes 120, 130 are in contact with the skin in cymba concha and cavum concha during the duration of a taVNS session. In particular, the electrically conducting material, preferably electrically conducting metal or metal alloy, should not cause any significant skin irritation during such normal use of the taVNS system 100.

Currently preferred electrically conducting materials include aluminum and aluminum alloys. Aluminum has a resistivity of 2.65×10⁻⁸ Ωm and an electrical conductivity of 3.77×10⁷ S/m at 20°C.

In an embodiment, the cymba concha electrode 120 is made of aluminum or an aluminum alloy or comprises an electrode surface 121 made of aluminum or the aluminum alloy. Alternatively, or preferably in addition, the cavum concha electrode 130 is made of aluminum or an aluminum alloy or comprises an electrode surface 131 made of aluminum of the aluminum alloy. In a preferred embodiment, the cymba concha electrode 120 and the cavum concha electrode 130 are made of aluminum or comprise a respective electrode surface 121, 131 made of aluminum.

Other metals and metal alloys that could be used for the electrodes 120, 130 or at least the electrode surfaces 121, 131 include gold and silver and alloys thereof.

The electrodes 120, 130 may be made of a single metal or metal alloy, preferably aluminum or an aluminum alloy. For instance, the electrodes 120, 130 could be solid electrodes consisting of the single metal or metal alloy. Alternatively, the electrodes 120, 130 could comprise an electrically conducting core material, preferably an electrically conducting metal or metal alloy, and then comprising an electrode surface 121, 131 made of another electrically conducting material, preferably another electrically conducting metal or metal alloy. The electrode surface 121, 131 could then be in the form an electrically conducting coating or surface layer on the electrode core. As an example, the electrode core could be made of copper or an alloy thereof.

The electrically conducting material of the electrode core does then not necessarily have to be biologically acceptable since it will not be in direct contact with the skin of the human subject. Thus, electrically conducting material, preferably electrically conducting metals or metal alloys, having a high electrically conductivity but not necessary biologically acceptable could be used as electrode core material.

In a preferred embodiment, the cymba concha electrode 120 and the cavum concha electrode 130 are made of the same electrically conducting material or materials. Hence, both electrodes 120, 130 are preferably solid electrodes made of a single electrically conducting material, preferably a single electrically conducting metal or metal alloy, or both electrodes 120, 130 comprise an electrically conducting core material, preferably an electrically conducting metal or metal alloy, and an electrode surface 121, 131 made of another electrically conducting material, preferably another electrically conducting metal or metal alloy.

In an embodiment, the cymba concha electrode 120 is configured to be in direct physical contact with the skin in the cymba concha of the ear of the human subject and the cavum concha electrode 130 is configured to be in direct physical contact with the skin in the cavum concha of the ear of the human subject.

Hence, the two electrodes 120, 130 of the electrode support 110 are configured to be not only in electrical contact with but also direct physical contact with the skin in the cymba concha and cavum concha, respectively. In particular, the respective electrode surface 121, 131 of the electrodes 120, 130 is configured to be in direct physical contact and electrical contact with the skin in these regions (cymba concha and cavum concha, respectively) of the ear of the human subject.

Direct physical contact as used herein means that the electrodes 120, 130, or rather the electrode surface 121, 131, directly contacts and touches the skin in cymba concha and cavum concha, respectively. Accordingly, no intermediate medium, including an electrically conducting gel, is used to mediate the electrical contact between the electrodes 120, 130 and the skin.

In an embodiment, the cymba concha electrode 120 comprises an electrode body 123 in electrical contact with a power cord 150 and a hemispherical electrode head 122 comprising an electrode surface 121 configured to be in contact with the skin the cymba concha of the ear of the human subject.

In an embodiment, the electrode body 123 could be in the form of a cylinder having the hemispherical electrode head 122 at one of its ends and the other, opposite end facing the electrode support 110. Such a design of the cymba concha electrode 120 provides a smooth hemispherical electrode surface 121 touching the skin at cymba concha while the electrode body 123 enables the hemispherical electrode head 122 to protrude into cymba concha while avoiding surrounding structures or regions in the ear that should not be electrically stimulated, such as the crus of helix, antihelix and inferior crus of antihelix.

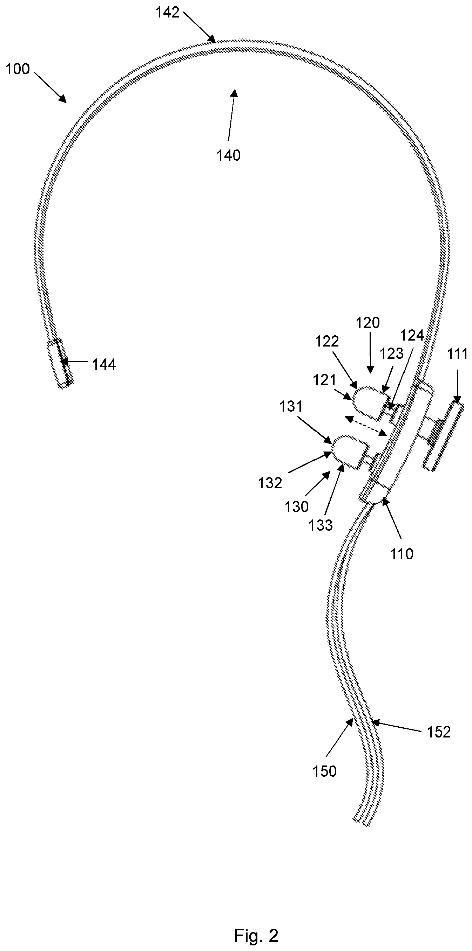

In an embodiment, the electrode body 123 of the cymba concha electrode 120 is attached to an adjustable electrode shaft 124, see Fig. 2. In such an embodiment, the adjustable electrode shaft 124 is arranged to adjust a protrusion of the hemispherical electrode head 122 of the cymba concha electrode 120 relative the electrode support 110.

In this embodiment, the protrusion of the cymba concha electrode 120 relative the electrode support 110 is adjustable as indicated by the hatched arrow in Fig. 2. This means that the distance between the end of the hemispherical electrode head 122 and thereby of the electrode surface 121 and the electrode support 110 could be adjusted so that electrode surface 121 correctly touches the skin in cymba concha when the elastic headband 140 is attached to the head of the human subject.

In a particular embodiment, the taVNS system 100 also comprises an adjustment screw 111 arranged at the electrode support 110 in contact with the adjustable electrode shaft 124. The adjustment screw 111 is then configured to attach the adjustable electrode shaft 124 at a selected position relative the electrode support 110.

In a preferred embodiment, the adjustment screw 111 can be screwed clockwise or counter-clockwise relative the electrode support 110. Turning the adjustment screw 111 one way, preferably clockwise, causes movement of the cymba concha electrode 120 further away from the electrode support 110, whereas turning the adjustment screw 111 the other way, preferably counter-clockwise, causes movement of the cymba concha electrode 120 towards the electrode support 110. Accordingly, the adjustment screw 111 can thereby be used to adjust the amount or level of protrusion of the cymba concha electrode 120, and in particular of the hemispherical electrode head 122, from the electrode support 110.

In an embodiment, the cavum concha electrode 130 comprises an electrode body 133 in electrical contact with a power cord 152 and a hemispherical electrode head 132 comprising an electrode surface 131 configured to be in contact with the skin the cavum concha of the ear of the human subject.

In an embodiment, the electrode body 133 could be in the form of a cylinder having the hemispherical electrode head 132 at one of its ends and the other, opposite end facing the electrode support 110. Such a design of the cymba concha electrode 130 provides a smooth hemispherical electrode surface 131 touching the skin at cavum concha while the electrode body 133 enables the hemispherical electrode head 132 to protrude into cavum concha while avoiding surrounding structures or regions in the ear that should not be electrically stimulated, such as the crus of helix, tragus, and antitragus.

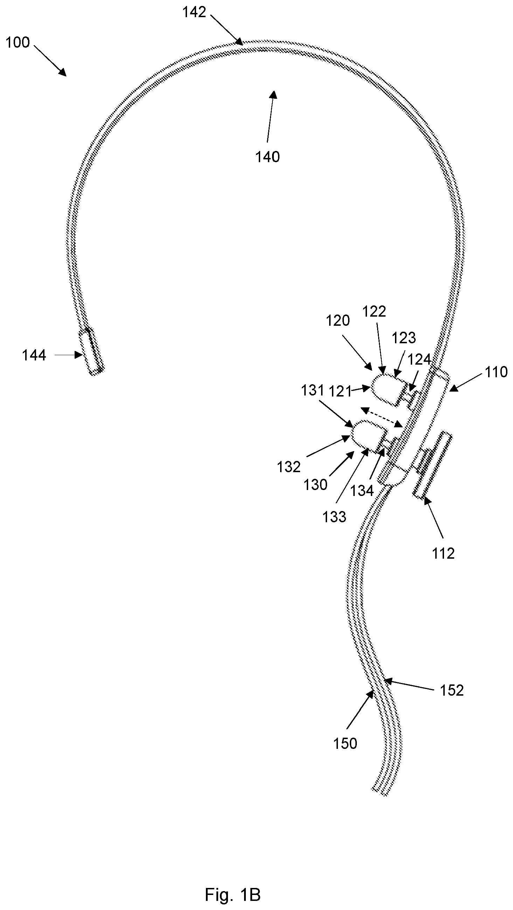

In an embodiment, the electrode body 133 of the cavum concha electrode 130 is attached to an adjustable electrode shaft 134, see Fig. 1B. In such an embodiment, the adjustable electrode shaft 134 is arranged to adjust a protrusion of the hemispherical electrode head 132 of the cavum concha electrode 130 relative the electrode support 110.

In this embodiment, the protrusion of the cymba concha electrode 130 relative the electrode support 110 is adjustable as indicated by the hatched arrow in Fig. 1B. This means that the distance between the end of the hemispherical electrode head 132 and thereby of the electrode surface 131 and the electrode support 110 could be adjusted so that electrode surface 131 correctly touches the skin in cavum concha when the elastic headband 140 is attached to the head of the human subject.

In a particular embodiment, the taVNS system 100 also comprises an adjustment screw 112 arranged at the electrode support 110 in contact with the adjustable electrode shaft 134. The adjustment screw 112 is then configured to attach the adjustable electrode shaft 134 at a selected position relative the electrode support 110.

In a preferred embodiment, the adjustment screw 112 can be screwed clockwise or counter-clockwise relative the electrode support 110. Turning the adjustment screw 112 one way, preferably clockwise, causes movement of the cavum concha electrode 130 further away from the electrode support 110, whereas turning the adjustment screw 112 the other way, preferably counter-clockwise, causes movement of the cavum concha electrode 130 towards the electrode support 110. Accordingly, the adjustment screw 112 can thereby be used to adjust the amount or level of protrusion of the cavum concha electrode 130, and in particular of the hemispherical electrode head 132, from the electrode support 110.

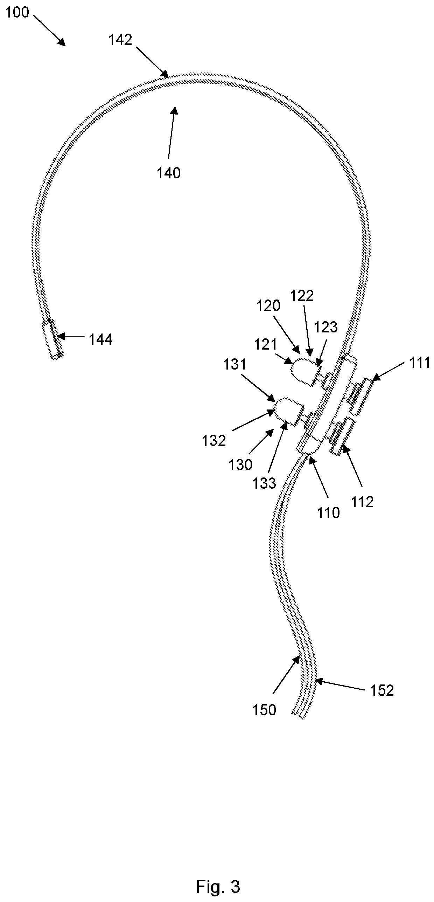

The embodiment shown in Figs. 1A-1C enables adjustment of the protrusion of the cavum concha electrode 130, whereas the embodiment shown in Fig. 2 enables adjustment of the protrusion of the cymba concha electrode 120. Fig. 3 illustrates another embodiment combining adjustment of the protrusion of the cavum concha 130 with adjustment of the protrusion of the cymba concha electrode 120.

The embodiment of the taVNS system 100 as shown in Fig. 3 provides the largest degree of freedom by individually adjusting the protrusion of the two electrodes 120, 130 relative the electrode support 110. However, it is in most cases sufficient if the protrusion of merely one of the electrodes 120, 130 is adjustable to still achieve efficient electrical and physical contact between the electrode surfaces 121, 131 and the skin in the cymba concha and cavum concha. In such a case, one of the electrodes 120, 130 is fixedly, i.e., non-adjustably, attached to the electrode support 110, whereas the other electrode 120, 130 is adjustably attached to the electrode support 110. Such an embodiment leads to a less complex design of the taVNS system 100 in Figs. 1A-1C or 2 as compared to the embodiment of the taVNS system 100 as shown in Fig. 3. In a currently preferred embodiment, the protrusion of the cavum concha electrode 130 is adjustable whereas the cymba concha electrode 120 is fixed as shown in Figs. 1A-1C.

The elastic headband 140 of the taVNS system 100 exerts an inwards pressure or force on the electrode support 110 that then presses the fixed electrode in physical contact with the skin at the target stimulation site in the external ear of the human subject. The amount or level of protrusion of the adjustable electrode can then be used to fine tune the pressure exerted by the adjustable electrode on the skin to achieve a direct physical contact between the adjustable electrode and the skin but not being uncomfortable for the human subject. In other words, the design of the taVNS system 100 with the elastic headband 140 attached to the electrode support 110 enables sufficient electrical and physical contact between both electrodes 120, 130 and the skin at cymba concha and cavum concha and still not pressing the electrodes 120, 130 too tightly against the skin to be uncomfortable for the human subject. This is possible even with one fixed electrode and one adjustable electrode.

Hence, in an embodiment, one of the cymba concha electrode 120 and the cavum concha 130 is fixedly attached to the electrode support 110 and protrudes at a fixed distance from the electrode support 110. In this embodiment, the other of the cymba concha electrode 120 and the cavum concha 130 is adjustably attached to the electrode support 110 and protrudes at an adjustable distance from the electrode support 110.

In an embodiment, the distance between the cymba concha electrode 120 and the cavum concha electrode 130 in the electrode support 110 corresponds to an average distance between the cymba concha and the cavum concha in a population of a plurality of human subjects.

The inventors have found that the distance between the cymba concha and the cavum concha does not differ much in a population of human adults. This means that an electrode support 110 with a fixed, i.e., non-adjustable, distance between the cymba concha electrode 120 and the cavum concha electrode 130 could be used to electrically stimulate a plurality of human subjects. Hence, taVNS system 100 can still be used to treat a plurality of different human (adult) subjects without the need for adjusting the distance between the electrodes 120, 130 in the electrode support 110.

In another embodiment, the distance between the cymba concha electrode 120 and the cavum concha electrode 130 in the electrode support 110 corresponds to a distance between the cymba concha and the cavum concha of a human subject.

In this embodiment, a subject-specific taVNS system 100 could be used, in which the distance between the cymba concha electrode 120 and the cavum concha electrode 130 in the electrode support 110 is defined to match the corresponding distance between the cymba concha and the cavum concha of the particular human subject.

In a particular embodiment, distance between the cymba concha electrode 120 and the cavum concha electrode 130 in the electrode support 110 corresponds to a distance along the length (L) or length axis of the electrode support 110, see Fig. 1C.

The electrode support 110 of the taVNS system 100 is designed to be positioned external of the outer ear of the human subject when the electrodes 120, 130 are in contact with the skin of the cymba concha and cavum concha, respectively. In a typical embodiment, the electrode support 110 is configured to be aligned with at least a portion of the external ear of the human subject when the elastic headband 140 is attached to the head of the human subject. However, the electrode support 110 is preferably present outside of the external ear, i.e., not in the form of an ear shell to be attached inside the external ear.

In an embodiment, the electrodes 120, 130 are attached to and protrude from an inner surface 113 of the electrode support 110, see Fig. 1C. This inner surface 113 of the electrode support 110 is thereby facing the external ear of the human subject when the elastic headband 140 is attached to the head of the human subject. The inner surface 113 is preferably a flat surface, i.e., does not correspond to a surface of the ear.

The taVNS system 100 comprises an elastic headband 140 attached to the electrode support 110. The elastic headband 140 is designed to be attached to the head of the human subject. At such a position on the head, the electrode support 110 will be aligned with one external or outer ear of the human subject, i.e., the right ear or the left ear, preferably the left ear. Furthermore, at such a position the cymba concha electrode 120 will be aligned with the cymba concha and the cavum concha electrode 130 will be aligned with cavum concha of the ear. The headband 140 is elastic to enable it to be attached to the head of the human subject but is preferably made of or comprises a rigid material that is capable of exerting an inward pressure or force on the electrode support 110 against the ear of the human subject.

The headband 140 comprises an over-head band or piece 142 designed to run over and on top of a least a portion of the head of the human subject. The headband 140 may optionally be held in position against the head of the human subject by a retention device 144. In such a case, the retention device 144 is preferably arranged at one end of the over-head band 142 with the electrode support 110 arranged at the other end of the over-head band 142. The retention device 144 will not only provide a comfortable contact with the side of the head preferably at a position above the non-stimulated ear but may also help balance the headband 140 on the head of the human subject. Hence, the retention device 144 could provide a counterweight to at least partly balance the weight of the electrode support 110.

The elastic headband 140 may be attached to the electrode support 110 or may be integrally formed with the electrode support 110. In the latter case, the elastic headband 140, or at least the over-head band 142 thereof, and the electrode support 110 forms a monolithic unit.

The headband 140 may optionally include an adjustment mechanism for fitting the headband 140 to the head of the human subject. Such an adjustment mechanism can then be used to adjust the size of the headband 140 to different head sizes. Furthermore, the adjustment mechanism could be used to adjust the pressure exerted by the headband 140 onto the electrode support 110 to press the electrodes 120, 130 attached to and protruding from the electrode support 110 in physical contact with the skin in the cymba concha and cavum concha of the ear. An illustrative, but non-limiting, example of such an adjustment mechanism is to include telescoping sections to extend or retract the ends of the elastic headband 140. Such telescoping sections could then be lockable using well-known solutions, such as friction or rachet brakes.

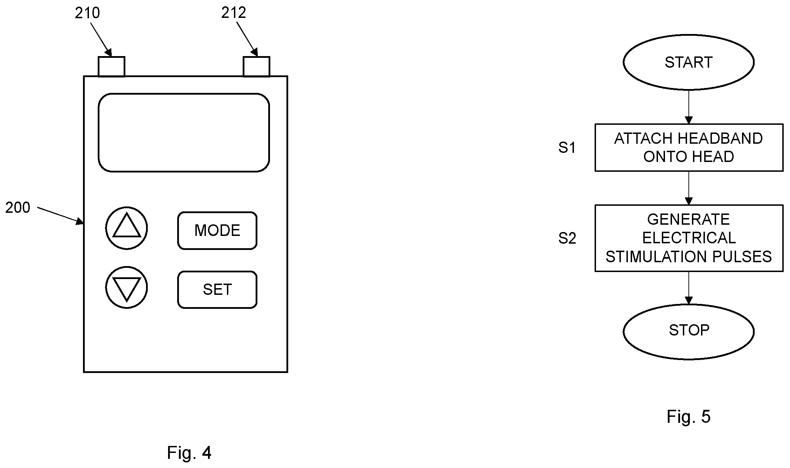

In an embodiment, the taVNS system 100 also comprises a stimulation device 200, see Fig. 4. In such a case, the stimulation device 200 is in electrical contact with the cymba concha electrode 120 and the cavum concha electrode 130. The stimulation device 200 is configured to generate electrical stimulation pulses.

The stimulation device 200 typically contains connection ports 210, 212, to which the above-mentioned power cords 150, 152 could be attached to electrically connect the stimulation device 200 with the electrodes 120, 130.

The stimulation device 200 could be any stimulation device capable of generating electrical stimulation pulses suitable for taVNS. Such stimulation devices 200, sometimes referred to as transcutaneous electrical nerve stimulation (TENS) devices or machines, are available on the marked from various vendors including, but not limited to, TENS 7000^{®}, Omron, Axion, Body Clock, Saneo, Health Technologies, Roscoe Medical, Auri Stim Medical, tVNS Technology GmbH, Cerbomed, Parasym, Soterix Medical, Xana Stim, NESOS, etc.

Fig. 5 is a flow chart of a method for transcutaneous auricular vagus nerve stimulation (taVNS) in a human subject according to an embodiment. The method comprises attaching, in step S1, the elastic headband 140 of the taVNS system 100 according to the invention onto a head of the human subject to position the cymba concha electrode 120 in contact with skin in cymba concha of an ear of the human subject and the cavum concha electrode 130 in contact with skin in cavum concha of the ear of the human subject. The method also comprises generating, in step S2, electrical stimulation pulses by the stimulation device 200 to apply the electrical stimulation pulses over the cymba concha electrode 120 and the cavum concha electrode 130.

An advantage of the present taVNS system 100 is that comparatively short stimulation sessions can be used and still achieving biological effects on the human subject as shown in the Example section. Hence, in an embodiment, step S2 in Fig. 5 comprises generating the electrical stimulation pulses by the stimulation device 200 once or twice, preferably twice, a day for a respective stimulation period or session of from 2 minutes up to 10 minutes, preferably from 3 minutes up to 7 minutes, and more preferably for about 5 minutes.

The taVNS system 100 of the invention and the use thereof as shown in Fig. 5 could be applied to treat various medical conditions, which would benefit from taVNS. Illustrative, but non-limiting, examples of such medical conditions include epilepsy, depression, obesity, migraine, cluster headaches, insomnia, neuropathic pain, back pain, chronic pain, cognitive decline, inflammatory diseases, such as rheumatoid arthritis, Crohn's disease, ulcerative colitis, systemic lupus erythematosus (SLE), multiple sclerosis and Alzheimer's disease.

### EXAMPLES

The present Example investigated the effects of taVNS on cytokine levels in LPS-activated whole blood cultures from healthy donors before and after taVNS.

### Materials & Methods

### Study design

The study enrolled 13 healthy individuals between 21 and 75 years of age. Participants confirmed no non-steroidal anti-inflammatory drug (NSAID) medications within 48 hours and no food or drink (besides water) for at least 8 h prior to the study. The study schedule for each participant was a baseline pre-stimulation blood draw, a 30-minute wait period, 5 minutes of stimulation with the taVNS device of the invention (with the following settings: frequency 20 Hz, pulse width 200 µs, current intensity individually adjusted below pain threshold denoting ≤ 3 mA) and 2 hours post stimulation blood draw. The study was arranged over three consecutive days with the baseline pre-stimulation blood draw for all patients taken before 10 am. All blood draws were collected into 10 mL sodium heparin tubes and assayed with the custom *ex vivo* whole blood endotoxin assay. One participant did not finish the study due to a failed post stimulation blood draw.

### Ex vivo endotoxin whole blood assay

Blood drawn in heparin tubes was immediately aliquoted and stimulated (hold time did not exceed 1 hour). All preparations were done in a biosafety level 2 (BSL-2) lab setting. In brief, endotoxin (lipopolysaccharide (LPS), *Escherichia coli* 0111:B4, Sigma Aldrich, USA) was re-suspended to 5 mg/ml, sonicated for 30 min, vortexed, and diluted with phosphate buffered saline (PBS) to generate a working 1 mg/ml stock stored at -20 °C. For each day of the study, fresh dilutions of LPS were made from this working stock. Working stock was sonicate for 30 minutes and serially diluted 10-fold with saline to concentrations of 50 µg/mL to 5 ng/mL. Heparinized blood was aliquoted into 15 round bottom 2 mL tubes and 10 µL of LPS dilutions were added to blood samples in triplicate to achieve final concentrations of 0 ng/mL and 10 ng/mL in 500 µL blood aliquots. These tubes were then incubated on an orbital rocking platform at 37 °C for 4 h. After 4 hours, plasma was collected from all samples by centrifugation for 10 minutes at 2000 × g in a refrigerated centrifuge and frozen at -20 °C for future analysis.

### Cytokine assays

Tumor necrosis factor (TNF) levels in the plasma were quantitated using a commercial enzyme-linked immunosorbent assay (ELISA) (R&D Systems #DY210) with all samples assayed in duplicate. Further cytokine analysis was performed on the blood stimulated with 0 ng/mL and 10 ng/mL LPS and these samples were assayed in duplicate on a custom MesoScale Discovery U-Plex cytokine panel for interleukin-1 receptor antagonist (IL-1RA), IL-1α, IL-1β, IL-6 and IL-8. Plate was imaged using MESO QuickPlex SQ 120 instrument (MSD, Gaithersburg, MD) and analyzed using MSD Discovery Workbench Software. All data was analyzed and graphed using GraphPad Prism 9.

### Statistics

**Table 1 - Statistics**

| **LPS 10 ng/mL** | **Is the data paired?** | **Is the data normal? (Shapiro-Wilk)** | **Test** |
|---|---|---|---|
| IL-1RA | Yes | Yes | Paired T-Test |
| IL-1α | Yes | Yes | Paired T-Test |
| IL-1β | Yes | No | Wilcoxon Test |
| IL-6 | Yes | No | Wilcoxon Test |
| IL-8 | Yes | No | Wilcoxon Test |
| TNF | Yes | No | Wilcoxon Test |

### Results

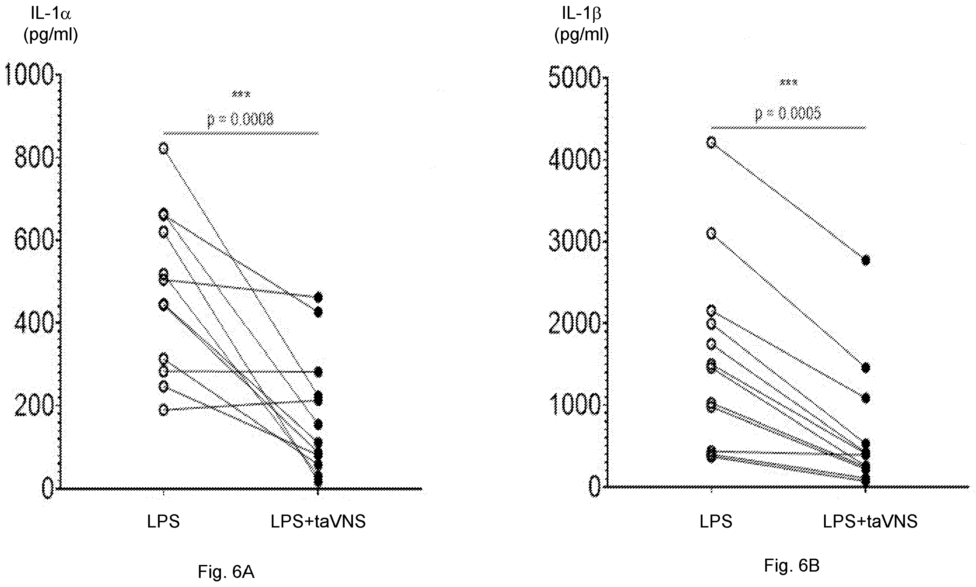

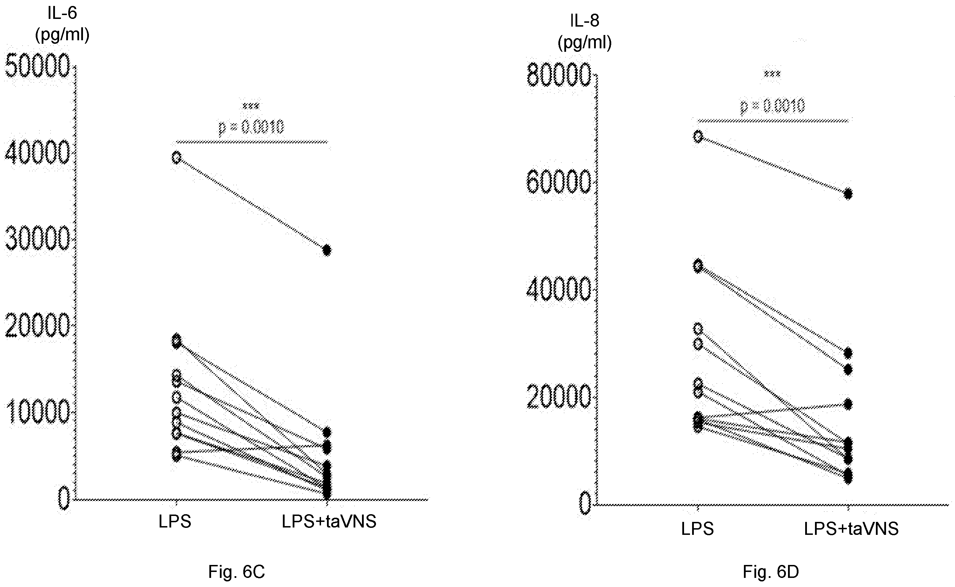

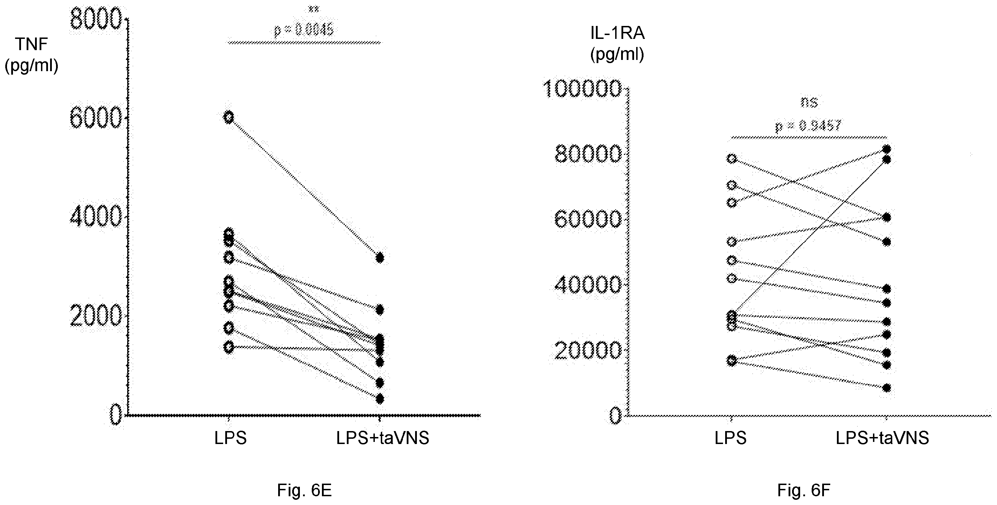

The results are presented in Figs. 6A-6F demonstrating the effects of taVNS on cytokine levels in LPS-activated (10 ng/mL) whole blood cultures from healthy donors before and after taVNS. Quantitative assessments were performed of the pro-inflammatory cytokines IL-1α (Fig. 6A), IL-1β (Fig. 6B), IL-6 (Fig. 6C), IL-8 (Fig. 6D), and TNF (Fig. 6E) and the anti-inflammatory cytokine IL-1RA (Fig. 6F). As is shown in the figures, taVNS stimulation induced a reduction in the investigated pro-inflammatory cytokines but had no significant effect on the anti-inflammatory cytokine IL-1RA. This Example thereby showed that the taVNS system of the invention was capable of effective taVNS stimulation, which reduced inflammatory cytokines. Hence, the taVNS system of the invention would be suitable for treatment of various inflammatory diseases and conditions that would benefit from taVNS stimulation.

## Claims

1. A transcutaneous auricular vagus nerve stimulation, taVNS, system (100) comprising:
an electrode support (110);
a cymba concha electrode (120) attached to and protruding from the electrode support (110) and configured to be in contact with skin in cymba concha of an ear of a human subject;
a cavum concha electrode (130) attached to and protruding from the electrode support (110) and configured to be in contact with skin in cavum concha of the ear of the human subject; and
an elastic headband (140) attached to the electrode support (110) and configured, when attached to a head of the human subject, to exert a pressure onto the electrode support (110) to press the cymba concha electrode (120) towards the skin in cymba concha of the ear of the human subject and the cavum concha electrode (130) onto the skin in cavum concha of the ear of the human subject, wherein
the electrode support (110) does not comprise any other electrodes than the cymba concha electrode (120) and the cavum concha electrode (130); **characterized in that**
one of the cymba concha electrode (120) and the cavum concha electrode (130) is adjustably attached to the electrode support (110) and protrudes at an adjustable distance from the electrode support (110), and **in that** the other of the cymba concha electrode (120) and the cavum concha electrode (130) is fixedly attached to the electrode support (110) and protrudes at a fixed distance from the electrode support (110).

2. The taVNS system according to claim 1, wherein the cymba concha electrode (120) is made of aluminum or an aluminum alloy, preferably aluminum, or comprises an electrode surface (121) made of aluminum or the aluminum alloy, preferably aluminum.

3. The taVNS system according to claim 1 or 2, wherein the cavum concha electrode (130) is made of aluminum or an aluminum alloy, preferably aluminum, or comprises an electrode surface (131) made of aluminum or the aluminum alloy, preferably aluminum.

4. The taVNS system according to any one of claim 1 to 3, wherein
the cymba concha electrode (120) is configured to be in direct physical contact with the skin in the cymba concha of the ear of the human subject; and
the cavum concha electrode (130) is configured to be in direct physical contact with the skin in the cavum concha of the ear of the human subject.

5. The taVNS system according to any one of claims 1 to 4, wherein the cymba concha electrode (120) comprises:
an electrode body (123) in electrical contact with a power cord (150); and
a hemispherical electrode head (122) comprising an electrode surface (121) configured to be in contact with the skin in the cymba concha of the ear of the human subject.

6. The taVNS system according to claim 5, wherein the electrode body (123) of the cymba concha electrode (120) is attached to an adjustable electrode shaft (124) arranged to adjust a protrusion of the hemispherical electrode head (122) of the cymba concha electrode (120) relative the electrode support (110).

7. The taVNS system according to claim 6, further comprising an adjustment screw (111) arranged at the electrode support (110) in contact with the adjustable electrode shaft (124) and configured to attach the adjustable electrode shaft (124) at a selected position relative the electrode support (110).

8. The taVNS system according to any one of claims 5 to 7, wherein the electrode surface (121) of the hemispherical electrode head (122) of the cymba concha electrode (120) is configured to be in direct physical contact and electrical contact with skin in the cymba concha of the ear of the human subject.

9. The taVNS system according to any one of claims 1 to 4, wherein the cavum concha electrode (130) comprises:
an electrode body (133) in electrical contact with a power cord (152); and
a hemispherical electrode head (132) comprising an electrode surface (131) configured to be in contact with the skin in the cavum concha of the ear of the human subject.

10. The taVNS system according to claim 9, wherein the electrode body (133) of the cavum concha electrode (130) is attached to an adjustable electrode shaft (134) arranged to adjust a protrusion of the hemispherical electrode head (132) of the cavum concha electrode (130) relative the electrode support (110).

11. The taVNS system according to claim 10, further comprising an adjustment screw (112) arranged at the electrode support (110) in contact with the adjustable electrode shaft (134) and configured to attach the adjustable electrode shaft (134) at a selected position relative the electrode support (110).

12. The taVNS system according to any one of claims 9 to 11, wherein the electrode surface (131) of the hemispherical electrode head (132) of the cavum concha electrode (130) is configured to be in direct physical contact and electrical contact with skin in the cavum concha of the ear of the human subject.

13. The taVNS system according to any one of claims 1 to 12, wherein a distance between the cymba concha electrode (120) and the cavum concha electrode (130) in the electrode support (110) corresponds to an average distance between the cymba concha and the cavum concha in a population of a plurality of human subjects.

14. The taVNS system according to any one of claims 1 to 13, wherein the cymba concha electrode (120) and the cavum concha electrode (130) are attached to and protrude from an inner surface (113) of the electrode support (110), wherein the inner surface (113) is a flat surface.

15. The taVNS system according to any one of claims 1 to 14, further comprising a stimulation device (200) in electrical contact with the cymba concha electrode (120) and the cavum concha electrode (130) and configured to generate electrical stimulation pulses.

## Patentansprüche

1. System (100) zur transkutanen aurikulären Vagusnervstimulation, taVNS-System, umfassend:
einen Elektrodenträger (110);
eine Cymba-Concha-Elektrode (120), die an dem Elektrodenträger (110) befestigt ist und von diesem hervorsteht und dazu konfiguriert ist, mit der Haut in der *Cymba conchae* eines Ohrs eines menschlichen Subjekts in Kontakt zu stehen;
eine Cavum-Concha-Elektrode (130), die an dem Elektrodenträger (110) befestigt ist und von dieser hervorsteht und dazu konfiguriert ist, mit der Haut im *Cavum conchae* des Ohrs des menschlichen Subjekts in Kontakt zu stehen; und
einen elastischen Kopfbügel (140), der an dem Elektrodenträger (110) befestigt und dazu konfiguriert ist, wenn er an einem Kopf des menschlichen Subjekts befestigt ist, einen Druck auf den Elektrodenträger (110) auszuüben, um die Cymba-Concha-Elektrode (120) in Richtung der Haut in der *Cymba conchae* des Ohrs des menschlichen Subjekts und die Cavum-Concha-Elektrode (130) auf die Haut im *Cavum conchae* des Ohrs des menschlichen Subjekts zu drücken, wobei
der Elektrodenträger (110) keine anderen Elektroden als die Cymba-Concha-Elektrode (120) und die Cavum-Concha-Elektrode (130) umfasst; **dadurch gekennzeichnet, dass**
eine der Cymba-Concha-Elektrode (120) und der Cavum-Concha-Elektrode (130) einstellbar an dem Elektrodenträger (110) befestigt ist und in einem einstellbaren Abstand von dem Elektrodenträger (110) vorsteht, und dadurch, dass die andere der Cymba-Concha-Elektrode (120) und der Cavum-Concha-Elektrode (130) fest an dem Elektrodenträger (110) befestigt ist und in einem festen Abstand von dem Elektrodenträger (110) vorsteht.

2. taVNS-System nach Anspruch 1, wobei die Cymba-Concha-Elektrode (120) aus Aluminium oder einer Aluminiumlegierung, vorzugsweise Aluminium, hergestellt ist oder eine Elektrodenoberfläche (121) umfasst, die aus Aluminium oder der Aluminiumlegierung, vorzugsweise Aluminium, hergestellt ist.

3. taVNS-System nach Anspruch 1 oder 2, wobei die Cavum-Concha-Elektrode (130) aus Aluminium oder einer Aluminiumlegierung, vorzugsweise Aluminium, hergestellt ist oder eine Elektrodenoberfläche (131) umfasst, die aus Aluminium oder der Aluminiumlegierung, vorzugsweise Aluminium, hergestellt ist.

4. taVNS-System nach einem der Ansprüche 1 bis 3, wobei
die Cymba-Concha-Elektrode (120) dazu konfiguriert ist, in direktem physischen Kontakt mit der Haut in der *Cymba conchae* des Ohrs des menschlichen Subjekts zu stehen; und
die Cavum-Concha-Elektrode (130) dazu konfiguriert ist, in direktem physischen Kontakt mit der Haut in dem *Cavum conchae* des Ohrs des menschlichen Subjekts zu stehen.

5. taVNS-System nach einem der Ansprüche 1 bis 4, wobei die Cymba-Concha-Elektrode (120) Folgendes umfasst:
einen Elektrodenkörper (123) in elektrischem Kontakt mit einem Stromkabel (150); und
einen halbkugelförmigen Elektrodenkopf (122), umfassend eine Elektrodenoberfläche (121), die dazu konfiguriert ist, mit der Haut in der *Cymba conchae* des Ohrs des menschlichen Subjekts in Kontakt zu stehen.

6. taVNS-System nach Anspruch 5, wobei der Elektrodenkörper (123) der Cymba-Concha-Elektrode (120) an einem einstellbaren Elektrodenschaft (124) befestigt ist, der angeordnet ist, um einen Vorsprung des halbkugelförmigen Elektrodenkopfes (122) der Cymba-Concha-Elektrode (120) relativ zu dem Elektrodenträger (110) einzustellen.

7. taVNS-System nach Anspruch 6, ferner umfassend eine Einstellschraube (111), die an dem Elektrodenträger (110) in Kontakt mit dem einstellbaren Elektrodenschaft (124) angeordnet und dazu konfiguriert ist, den einstellbaren Elektrodenschaft (124) an einer ausgewählten Position relativ zu dem Elektrodenträger (110) zu befestigen.

8. taVNS-System nach einem der Ansprüche 5 bis 7, wobei die Elektrodenoberfläche (121) des halbkugelförmigen Elektrodenkopfes (122) der Cymba-Concha-Elektrode (120) dazu konfiguriert ist, in direktem physikalischen Kontakt und elektrischen Kontakt mit der Haut in der *Cymba conchae* des Ohrs des menschlichen Subjekts zu stehen.

9. taVNS-System nach einem der Ansprüche 1 bis 4, wobei die Cavum-Concha-Elektrode (130) Folgendes umfasst:
einen Elektrodenkörper (133) in elektrischem Kontakt mit einem Stromkabel (152); und
einen halbkugelförmigen Elektrodenkopf (132), umfassend eine Elektrodenoberfläche (131), die dazu konfiguriert ist, mit der Haut in dem *Cavum conchae* des Ohrs des menschlichen Subjekts in Kontakt zu stehen.

10. taVNS-System nach Anspruch 9, wobei der Elektrodenkörper (133) der Cavum-Concha-Elektrode (130) an einem einstellbaren Elektrodenschaft (134) befestigt ist, der angeordnet ist, um einen Vorsprung des halbkugelförmigen Elektrodenkopfes (132) der Cavum-Concha-Elektrode (130) relativ zu dem Elektrodenträger (110) einzustellen.

11. taVNS-System nach Anspruch 10, ferner umfassend eine Einstellschraube (112), die an dem Elektrodenträger (110) in Kontakt mit dem einstellbaren Elektrodenschaft (134) angeordnet und dazu konfiguriert ist, den einstellbaren Elektrodenschaft (134) an einer ausgewählten Position relativ zu dem Elektrodenträger (110) zu befestigen.

12. taVNS-System nach einem der Ansprüche 9 bis 11, wobei die Elektrodenoberfläche (131) des halbkugelförmigen Elektrodenkopfes (132) der Cavum-Concha-Elektrode (130) dazu konfiguriert ist, in direktem physikalischen Kontakt und elektrischen Kontakt mit der Haut in dem *Cavum conchae* des Ohrs des menschlichen Subjekts zu stehen.

13. taVNS-System nach einem der Ansprüche 1 bis 12, wobei ein Abstand zwischen der Cymba-Concha-Elektrode (120) und der Cavum-Concha-Elektrode (130) in dem Elektrodenträger (110) einem durchschnittlichen Abstand zwischen der *Cymba conchae* und dem *Cavum conchae* in einer Population einer Vielzahl von menschlichen Subjekten entspricht.

14. taVNS-System nach einem der Ansprüche 1 bis 13, wobei die Cymba-Concha-Elektrode (120) und die Cavum-Concha-Elektrode (130) an einer Innenfläche (113) des Elektrodenträgers (110) befestigt sind und von dieser vorstehen, wobei die Innenfläche (113) eine flache Oberfläche ist.

15. taVNS-System nach einem der Ansprüche 1 bis 14, ferner umfassend eine Stimulationsvorrichtung (200) in elektrischem Kontakt mit der Cymba-Concha-Elektrode (120) und der Cavum-Concha-Elektrode (130) und das dazu konfiguriert ist, elektrische Stimulationsimpulse zu erzeugen.

## Revendications

1. Système de stimulation transcutanée du nerf vague auriculaire, taVNS, (100) comprenant :
un support d'électrodes (110) ;
une électrode de cymba concha (120) fixée à et faisant saillie à partir du support d'électrodes (110) et configurée pour être en contact avec la peau dans la cymba concha d'une oreille d'un sujet humain ;
une électrode de cavum concha (130) fixée à et faisant saillie à partir du support d'électrodes (110) et configurée pour être en contact avec la peau dans la cavum concha de l'oreille du sujet humain ; et
un bandeau élastique (140) fixé au support d'électrodes (110) et configuré, lors d'une fixation à une tête du sujet humain, pour l'exercice d'une pression sur le support d'électrodes (110) pour la compression de l'électrode de cymba concha (120) vers la peau dans la cymba concha de l'oreille du sujet humain et l'électrode de cavum concha (130) sur la peau dans la cavum concha de l'oreille du sujet humain, dans lequel
le support d'électrodes (110) ne comprend aucune autre électrode que l'électrode de cymba concha (120) et l'électrode de cavum concha (130) ; **caractérisé en ce que**
l'une de l'électrode de cymba concha (120) et de l'électrode de cavum concha (130) est fixée de manière ajustable au support d'électrodes (110) et fait saillie à une distance ajustable à partir du support d'électrodes (110), et **en ce que** l'autre de l'électrode de cymba concha (120) et de l'électrode de cavum concha (130) est fixée de manière fixe au support d'électrodes (110) et fait saillie à une distance fixe à partir du support d'électrodes (110).

2. Système taVNS selon la revendication 1, dans lequel l'électrode de cymba concha (120) est constituée d'aluminium ou d'un alliage d'aluminium, de préférence d'aluminium, ou comprend une surface d'électrode (121) constituée d'aluminium ou de l'alliage d'aluminium, de préférence d'aluminium.

3. Système taVNS selon la revendication 1 ou la revendication 2, dans lequel l'électrode de cavum concha (130) est constituée d'aluminium ou d'un alliage d'aluminium, de préférence d'aluminium, ou comprend une surface d'électrode (131) constituée d'aluminium ou de l'alliage d'aluminium, de préférence d'aluminium.

4. Système taVNS selon l'une quelconque des revendications 1 à 3, dans lequel
l'électrode de cymba concha (120) est configurée pour être en contact physique direct avec la peau dans la cymba concha de l'oreille du sujet humain ; et
l'électrode de cavum concha (130) est configurée pour être en contact physique direct avec la peau dans la cavum concha de l'oreille du sujet humain.

5. Système taVNS selon l'une quelconque des revendications 1 à 4, dans lequel l'électrode de cymba concha (120) comprend :
un corps d'électrode (123) en contact électrique avec un cordon d'alimentation (150) ; et
une tête d'électrode hémisphérique (122) comprenant une surface d'électrode (121) configurée pour être en contact avec la peau dans la cymba concha de l'oreille du sujet humain.

6. Système taVNS selon la revendication 5, dans lequel le corps d'électrode (123) de l'électrode de cymba concha (120) est fixé à une tige d'électrode ajustable (124) agencée pour l'ajustement d'une saillie de la tête d'électrode hémisphérique (122) de l'électrode de cymba concha (120) par rapport au support d'électrodes (110).

7. Système taVNS selon la revendication 6, comprenant en outre une vis d'ajustement (111) agencée au niveau du support d'électrodes (110) en contact avec la tige d'électrode ajustable (124) et configurée pour la fixation de la tige d'électrode ajustable (124) à une position sélectionnée par rapport au support d'électrodes (110).

8. Système taVNS selon l'une quelconque des revendications 5 à 7, dans lequel la surface d'électrode (121) de la tête d'électrode hémisphérique (122) de l'électrode de cymba concha (120) est configurée pour être en contact physique direct et en contact électrique avec la peau dans la cymba concha de l'oreille du sujet humain.

9. Système taVNS selon l'une quelconque des revendications 1 à 4, dans lequel l'électrode de cavum concha (130) comprend :
un corps d'électrode (133) en contact électrique avec un cordon d'alimentation (152) ; et
une tête d'électrode hémisphérique (132) comprenant une surface d'électrode (131) configurée pour être en contact avec la peau dans la cavum concha de l'oreille du sujet humain.

10. Système taVNS selon la revendication 9, dans lequel le corps d'électrode (133) de l'électrode de cavum concha (130) est fixé à une tige d'électrode ajustable (134) agencée pour l'ajustement d'une saillie de la tête d'électrode hémisphérique (132) de l'électrode de cavum concha (130) par rapport au support d'électrodes (110).

11. Système taVNS selon la revendication 10, comprenant en outre une vis d'ajustement (112) agencée au niveau du support d'électrodes (110) en contact avec la tige d'électrode ajustable (134) et configurée pour la fixation de la tige d'électrode ajustable (134) à une position sélectionnée par rapport au support d'électrodes (110).

12. Système taVNS selon l'une quelconque des revendications 9 à 11, dans lequel la surface d'électrode (131) de la tête d'électrode hémisphérique (132) de l'électrode de cavum concha (130) est configurée pour être en contact physique direct et en contact électrique avec la peau dans la cavum concha de l'oreille du sujet humain.

13. Système taVNS selon l'une quelconque des revendications 1 à 12, dans lequel une distance entre l'électrode de cymba concha (120) et l'électrode de cavum concha (130) dans le support d'électrodes (110) correspond à une distance moyenne entre la cymba concha et la cavum concha dans une population d'une pluralité de sujets humains.

14. Système taVNS selon l'une quelconque des revendications 1 à 13, dans lequel l'électrode de cymba concha (120) et l'électrode de cavum concha (130) sont fixées à et font saillie à partir d'une surface interne (113) du support d'électrodes (110), dans lequel la surface interne (113) est une surface plate.

15. Système taVNS selon l'une quelconque des revendications 1 à 14, comprenant en outre un dispositif de stimulation (200) en contact électrique avec l'électrode de cymba concha (120) et l'électrode de cavum concha (130) et configuré pour la génération d'impulsions de stimulation électrique.
